Europäisches Patentamt

European Patent Office

Office européen des brevets

(11) Veröffentlichungsnummer: **0 607 821 A1**

## EUROPÄISCHE PATENTANMELDUNG

(21) Anmeldenummer: **94100196.8**

(51) Int. Cl.5: **C07D 209/48**

(22) Anmeldetag: **07.01.94**

(30) Priorität: **16.01.93 DE 4301024**

(43) Veröffentlichungstag der Anmeldung:
**27.07.94 Patentblatt 94/30**

(84) Benannte Vertragsstaaten:
**DE FR GB IT NL**

(71) Anmelder: **HOECHST AKTIENGESELLSCHAFT**
**Brüningstrasse 50**
**D-65929 Frankfurt am Main(DE)**

(72) Erfinder: **Jaekel, Frank, Dr.**
**Am Carlusbaum 24**
**D-65812 Bad Soden/Ts.(DE)**

(54) Verfahren zur Herstellung von gereinigten Phthalimidoalkancarbonsäuren.

(57) Die vorliegende Erfindung betrifft ein Verfahren zur Herstellung von gereinigten Phthalimidoalkancarbonsäuren, die mit Phthalsäure, Lactam und/oder Wasser verunreinigt sind, umfassend die Maßnahmen:
a) Aufschmelzen der verunreinigten Phthalimidoalkancarbonsäure,
b) Halten der Schmelze für eine so lange Zeit und bei einer solchen Temperatur, so daß sich die Verunreinigungen im wesentlichen aus der Schmelze entfernt haben und
c) Abkühlen der gereinigten Produktschmelze.

EP 0 607 821 A1

Phthalimidoalkancarbonsäuren dienen als Ausgangsstoffe für die entsprechenden Persäuren, die als Oxidationsmittel in Reinigungs-, Blech-, Desinfektions- und Oxidationsmitteln eingesetzt werden.

Die Phthalimidoalkanpercarbonsäuren zeichnen sich neben guten Bleicheigenschaften auch durch eine gute Lagerstabilität aus, die auch ohne Zusätze (Phlegmatisierung) oder Verdünnen mit Inertstoffen, wie Phosphinoxid/Natriumsulfat` Borsäure oder Magnesiumsulfat, erreicht wird. Aufgrund dieses Eigenschaftprofils erlangen Phthalimidoalkanpercarbonsäuren eine zunehmende Bedeutung als Bleichsysteme in Waschmitteln.

In der Fachliteratur sind zahlreiche Methoden zur Herstellung von Phthalimidoalkancarbonsäuren bekannt.

In Chem. Ber. Bd. 46, Seite 3158 (1913) wird die Herstellung von Phthalimidoalkancarbonsäuren durch Kondensation von Phthalsäureanhydrid und Aminosäuren beschrieben. Die hohen Preise der Aminosäuren stehen jedoch einer großtechnischen Verwirklichung im Wege.

Aus J. Am. Chem. Soc. 70, 2115 (1948) und J. Org. Chem. 24, 2062 (1959) ist die Herstellung von 6-Phthalimidohexansäure durch Erhitzen von $\epsilon$-Caprolactam mit Phthalsäureanhydrid auf eine Temperatur von 180 - 195°C bekannt. Das erhaltene Produkt wird entweder durch Destillation oder Umkristallisieren in Ausbeuten zwischen 65 und 80 % gewonnen. Bei diesem Verfahren kann zwar mit gut verfügbaren Ausgangsstoffen gearbeitet werden, die Ausbeuten sind aber für ein technisches Großprodukt unbefriedigend.

Die japanische Patentschrift Nr. Sho-46-21710 (Erteilungsdatum: 19. Juni 1971) offenbart die Herstellung von 4-Phthalimidobutansäure aus Phthalsäureanhydrid und Pyrrolidon in Gegenwart von 5 bis 15 % Wasser bei Temperaturen von 180°C und Reaktionszeiten von 5 Stunden. Um ein genügend reines Produkt zu erhalten, ist es erforderlich, das gewonnene Rohprodukt aus Methanol umzukristallisieren. Die Ausbeute liegt bei 86 %.

EP-A-0 349 940 nennt ein Verfahren zur Herstellung von Phthalimidoalkancarbonsäuren durch Umsetzung von Phthalsäureanhydrid und Lactamen, z. B. $\epsilon$-Caprolactam, in Gegenwart von Wasser bei einem Druck von 1 bis 30 bar, Temperaturen von 100 bis 250°C und Reaktionszeiten von 5 bis 20 Stunden.

Bei den vorstehend beschriebenen Verfahren enthalten die Phthalimidoalkancarbonsäuren vor ihrer Reinigung Verunreinigungen an Phthalsäure, Aminosäure bzw. Lactam und/oder Wasser.

Bei Einsatz von Phthalimidoalkancarbonsäuren als Ausgangsstoffe für die entsprechenden Phthalimidoalkanpercarbonsäuren sind derartige Verunreinigungen aus verschiedenen Gründen unerwünscht. Üblicherweise werden die Phthalimidoalkancarbonsäuren durch Umsetzung mit Wasserstoffperoxid in Gegenwart einer starken Säure zu den entsprechenden Persäuren oxidiert. Bei Anwesenheit von Phthalsäure reagiert diese zur Monoperoxophthalsäure, die im Gegensatz zu den Phthalimidoalkanpercarbonsäuren nur eine geringe Lagerstabilität aufweist und im Normalfall phlegmatisiert oder in das Magnesiumsalz überführt werden muß (vgl. EP-A-0 105 689). Phthalimidoalkanpercarbonsäuren, die mit unphlegmatisierter Monoperoxophthalsäure verunreinigt sind, würden einen Teil ihrer guten Lagerstabilität verlieren.

In EP-A-0 490 409 ist ein Verfahren zur Herstellung von Arylimidoalkanpercarbonsäuren beschrieben, bei dem Arylimidoalkancarbonsäuren in Methylenchlorid oder Chloroform gelöst werden und kontinuierlich mit Wasserstoffperoxid in Gegenwart einer starken Säure zu den entsprechenden Percarbonsäuren oxidiert werden. Zur bestmöglichen Verfahrensausführung ist es bei diesem Prozeß unerläßlich, daß die Arylimidoalkancarbonsäuren vollständig in Methylenchlorid bzw. Chloroform gelöst sind. Da weder Phthalsäure, Wasser noch Aminosäure bzw. Lactam sich in diesem Lösungsmittel lösen, müssen die eingesetzten Arylimidocarbonsäuren weitestgehend frei von diesen Verunreinigungen sein, will man nicht mit zusätzlichen Reinigungsschritten - wie Filtration oder Phasentrennung - die festen bzw. flüssigen Verunreinigungen abtrennen.

Aufgabe der vorliegenden Erfindung ist es, ein Verfahren zur Reinigung von Phthalimidoalkancarbonsäuren zur Verfügung zu stellen, das es ermöglicht, die vorhandenen Verunreinigungen an Phthalsäure, Lactam und/oder Wasser weitestgehend zu entfernen.

Gegenstand der Erfindung ist ein Verfahren zur Herstellung von gereinigten Phthalimidoalkancarbonsäuren, die mit Phthalsäure, Lactam und/oder Wasser verunreinigt sind, umfassend die Maßnahmen:

a) Aufschmelzen der verunreinigten Phthalimidoalkancarbonsäure,

b) Halten der Schmelze für eine so lange Zeit und bei einer solchen Temperatur, so daß sich die Verunreinigungen im wesentlichen aus der Schmelze entfernt haben und

c) Abkühlen der gereinigten Produktschmelze.

Bei den verwendeten ungereinigten Phthalimidoalkancarbonsäuren handelt es sich im allgemeinen um Produkte der bekannten Herstellungsverfahren. Überlicherweise enthalten diese verunreinigten Phthalimidoalkancarbonsäuren folgende Verunreinigungen:

1,5 - 10 Gew.-% Phthalsäure,

0,5 - 10 Gew.-% Lactam,

1,0 - 20 Gew.-% Wasser.

Zur Durchführung des erfindungsgemäßen Verfahrens gibt es verschiedene Ausführungsformen.

Zunächst wird die verunreinigte Phthalimidoalkancarbonsäure in einer geeigneten Apparatur, z.B. einem beheizbaren Rührkessel geschmolzen. Das Aufschmelzen erfolgt bevorzugt unter Begasung mit einem Inertgas, wie Argon, Stickstoff oder Kohlenstoffdioxid. Das Begasen kann auf verschiedene Arten erfolgen. So ist es vorstellbar, daß das Gas über die Schmelze geleitet wird, mit Hilfe einer Vorrichtung (z.B. "Lanze") in die Schmelze eingeblasen wird oder auf andere dem Fachmann bekannte Weise in den Rührkessel eingeleitet wird. Die Temperatur der Schmelze liegt bevorzugt zwischen 110 und 200°C, besonders bevorzugt 130 bis 170°C.

Anschließend wird der Inertgasdruck in der Apparatur durch Anlegen eines Vakuums gesenkt. Der Inertgasdruck in der Apparatur beträgt normalerweise 0,1 bis 300 mbar, bevorzugt 1 bis 140 mbar, besonders bevorzugt 4 bis 95 mbar. Der Zeitraum über den das Vakuum angelegt wird, ist abhängig von der gewünschten Reinheit der Phthalimidoalkancarbonsäure und der Temperatur der Rohproduktschmelze und beträgt im allgemeinen bis zu 5 Stunden. Es hat sich gezeigt, daß bei einer Temperatur der Schmelze von 140°C und einem Unterdruck von 105 mbar bereits nach weniger als 3 Stunden der Gehalt an Phthalsäure, ausgehend von 1,26 Gew.-%, auf weniger als 0,5 Gew.-% absinkt. Unter diesen Bedingungen fällt der Wassergehalt von 4,10 Gew.-% bereits nach einer halben Stunde auf unter 0,1 Gew.-%. Im allgemeinen gilt, daß höhere Temperaturen bzw. ein geringerer Druck die erforderliche Zeitdauer verkürzen, bzw. den Reinheitsgrad des Produktes erhöhen.

In einer weiteren Ausführungsform des erfindungsgemäßen Verfahrens wird das Rohprodukt in einer geeigneten Apparatur, z.B. einem druckstabilen heizbaren Rührkessel nach dem Evakuieren der Apparatur mit einem Inertgas begast und in der geschlossenen Apparatur geschmolzen. Der während des Aufheizens entstehende Überdruck wird von Zeit zu Zeit entspannt. Nach dem Entspannen wird erneut Inertgas in die Apparatur eingeleitet, die Apparatur druckdicht verschlossen und nach Einstellen eines Überdrucks wieder entspannt. Dieser Vorgang wird solange wiederholt, bis sich kein Überdruck mehr bildet.

Gegenüber diesem chargenweisen Verfahren wird jedoch das entsprechende kontinuierliche Verfahren bevorzugt.

Bei dem kontinuierlichen Verfahren wird das Rohprodukt in einer geeigneten Apparatur, z.B. einem druckstabilen heizbaren Rührkessel mit einem Inertgas begast und anschließend oder gleichzeitig geschmolzen. Das Begasen der Schmelze erfolgt bevorzugt durch Überleiten eines Inertgasstromes, wobei die Strömungsgeschwindigkeit des Inertgasstromes üblicherweise 0,1 bis 50 l/h, bevorzugt 1 bis 30 l/h, besonders bevorzugt 2 bis 20 l/h beträgt. Der Gasstrom kann bei Normaldruck oder auch einem leichten Unterdruck, bevorzugt im Bereich von 500-800 mbar in die Apparatur eingeleitet werden. Je nach Verunreinigung des Rohproduktes kann der Unterdruck auch geringer sein. Der Zeitraum währenddessen der Inertgasstrom über die Produktschmelze geleitet wird, beträgt normalerweise 0,1 bis 6 Stunden, bevorzugt 0,3 bis 3 Stunden, besonders bevorzugt 0,5 bis 1,5 Stunden. Dieser Zeitraum ist abhängig von der geforderten Reinheit, Temperatur der Produktschmelze und dem angelegten Unterdruck.

Im Anschluß an die vorstehend beschriebenen Maßnahmen b) wird die Schmelze abgekühlt und das Produkt der Weiterverarbeitung, z.B. Konfektionierung, zugeführt. Üblicherweise wird die Schmelze bis zum Festpunkt abgekühlt. Des weiteren ist es denkbar, die Schmelze bis unmittelbar oberhalb des Festpunktes abzukühlen, um sie einerseits leicht aus dem Reaktionsgefäß entfernen zu können und andererseits unverzüglich mit der Konfektionierung beginnen zu können.

Beispiele:

Die Gehalte an N,N-Phthaloylaminocapronsäure (PAC), Phthalsäure und $\epsilon$-Caprolactam werden mittels reversed Phase HPLC ermittelt. Die Restwassermengen werden nach Karl Fischer bestimmt.

Die Prozentangaben bedeuten Gewichtsprozent.

Beispiel 1

1 kg N,N-Phthaloylaminocapronsäure (PAC) mit den analytischen Daten:

| PAC: | 93,5 % |
|---|---|
| Phthalsäure: | 1,45 % |
| $\epsilon$-Caprolactam: | 0,55 % |
| Wasser: | 4,40 % |

werden in einem Kolben auf 155°C erhitzt. Sobald diese Temperatur erreicht ist, wird ein schwaches Vakuum von 105 mbar angelegt und die Schmelze für 5 Stunden bei dieser Temperatur gehalten. Nach jeweils einer Stunde werden Proben aus der Schmelze entnommen und der Gehalt der Proben an PAC, Phthalsäure, $\epsilon$-Caprolactam und Wasser bestimmt (Tabelle 1). Es wird ein unverfärbtes gereinigtes Endprodukt erhalten.

Tabelle 1:    (* Doppelbestimmungen)

| Probennahme nach: | PAC* | Phthalsäure* | $\epsilon$-Caprolactam* | Wasser* |
|---|---|---|---|---|
| 0 h (Anfangswert) | 93,50 % | 1,45 % | 0,55 % | 4,40 % |
| 1 h | 97,60 % | 1,06 % | 0,42 % | 0,10 % |
| 2 h | 97,86 % | 0,66 % | 0,35 % | 0,05 % |
| 3 h | 98,95 % | 0,39 % | 0,28 % | 0,04 % |
| 4 h | 98,90 % | 0,12 % | 0,22 % | 0,06 % |
| 5 h | 99,45 % | 0,09 % | 0,18 % | 0,05 % |

Beispiel 2

1 kg PAC mit den analytischen Daten:

| PAC: | 94,1 % |
|---|---|
| Phthalsäure: | 1,26 % |
| $\epsilon$-Caprolactam: | 0,50 % |
| Wasser: | 4,10 % |

werden in einem Kolben auf 170°C erhitzt. Sobald die Temperatur erreicht ist, wird ein Vakuum von 25 mbar angelegt und die Schmelze für 3 Stunden bei dieser Temperatur gehalten. Nach jeweils 0,5 bzw. 1 Stunde werden Proben aus der Schmelze entnommen und der Gehalt der Proben an PAC, Phthalsäure, $\epsilon$-Caprolactam und Wasser bestimmt (Tabelle 2). Es ist ein fast weißes gereinigtes Endprodukt erhalten.

# EP 0 607 821 A1

Tabelle 2

| Probennahme nach | PAC* | Phthalsäure* | $\epsilon$-Caprolactam* | Wasser* |
|---|---|---|---|---|
| 0 h (Anfangswert) | 94,10 % | 1,26 % | 0,50 % | 4,10 % |
| 0,5 h | 98,27 % | 1,06 % | 0,40 % | 0,21 % |
| 1 h | 98,62 % | 0,89 % | 0,32 % | 0,10 % |
| 1,5 h | 98,95 % | 0,68 % | 0,23 % | 0,07 % |
| 2 h | 99,34 % | 0,33 % | 0,18 % | 0,08 % |
| 3 h | 99,55 % | 0,18 % | 0,15 % | 0,04 % |

* Doppelbestimmungen

Beispiel 3

2 kg PAC mit den analytischen Daten:

| | |
|---|---|
| PAC: | 94,10 % |
| Phthalsäure: | 1,45 % |
| Wasser: | 4,40 % |

werden in einem Kolben mit Stickstoff überlagert und auf 155 °C erhitzt. Sowie die Temperatur erreicht ist, wird ein leichter Stickstoffstrom (2 - 10 l/h) über das inzwischen geschmolzene Produkt geleitet. Nach jeweils 15 bzw. 30 Minuten werden Proben entnommen und auf ihre Gehalte an PAC, Phthalsäure und Wasser untersucht (Tabelle 3). Als Endprodukt wird ein entwässertes PAC erhalten.

Tabelle 3

| Probennahme nach | PAC* | Wassergehalt* | Phthalsäure* |
|---|---|---|---|
| 0 min (Anfangswert) | 94,1 % | 4,40 % | 1,45 % |
| 15 min | 97,32 % | 1,15 % | 1,38 % |
| 30 min | 98,13 % | 0,35 % | 1,32 % |
| 60 min | 98,29 % | 0,10 % | 1,19 % |
| 90 min | 98,76 % | 0,12 % | 1,04 % |
| 120 min | 98,90 % | 0,10 % | 0,92 % |
| 150 min | 98,96 % | 0,15 % | 0,82 % |
| 180 min | 99,01 % | 0,0 % | 0,85 % |

* Doppelbestimmungen

Beispiel 4

In einem 350 l Kessel mit Rührer werden 45,2 kg $\epsilon$-Caprolactam, 59,2 kg Phthalsäureanhydrid und 7,2 kg entmineralisiertes Wasser vorgelegt. Der Kessel wird zweimal evakuiert, mit Stickstoff begast und druckdicht verschlossen. Nach dem Aufheizen ohne Rühren, wird die Reaktion unter Rühren bei 155 °C durchgeführt. Nach 10 Stunden Reaktionszeit wird der Wasserdampfüberdruck über ein Ventil in die Atmosphäre entspannt. Das Ventil wird wieder geschlossen und der Kessel erneut mit Stickstoff begast. Sobald sich ein Überdruck von 5 - 6 bar aufgebaut hat, wird wieder in die Atmosphäre entspannt. Nach

EP 0 607 821 A1

nochmaliger Wiederholung dieser Prozedur wird auf 120°C abgekühlt und das Produkt über eine Schuppenwalze ausgetragen. Als Endprodukt wird PAC in Form weißer Schuppen erhalten.

| Analytische Daten: | PAC: | 96,34 % |
|---|---|---|
| | Phthalsäure: | 1,33 % |
| | Wasser: | 1,45 %. |

Beispiel 5 (Vergleich zu Beispiel 4)

In einem 350 l Kessel mit Rührer werden 45,2 kg ε-Caprolactam, 59,2 kg Phthalsäureanhydrid und 7,2 kg entmineralisiertes Wasser vorgelegt. Der Kessel wird zweimal evakuiert, mit Stickstoff begast und druckdicht verschlossen. Nach dem Aufheizen ohne Rühren, wird die Reaktion unter Rühren bei 155°C durchgeführt, wobei der Innendruck bis auf maximal ca. 7,5 bar ansteigt. Nach 10 Stunden Reaktionszeit wird auf 120°C abgekühlt und das Produkt über eine Schuppenwalze ausgetragen. Man erhält PAC als weiße Schuppenware.

| Analytische Daten: | PAC: | 93,3 % |
|---|---|---|
| | Phthalsäure: | 1,45 % |
| | Wasser: | 5,0 %. |

Die Beispiele demonstrieren deutlich, wie auf einfache und effiziente Weise ein Rohprodukt der Imidocarbonsäure PAC gereinigt werden kann.

So ist in Beispiel 1 der Gehalt an Phthalsäure durch Anlegen eines nur schwachen Vakuums um 94 % reduziert worden, ε-Caprolactam um fast 70 %. Das Restwasser wurde praktisch vollkommen entfernt. Die Trocknung der Rohware PAC gelingt noch einfacher durch Überströmen der Produktschmelze mit Stickstoff oder wiederholtes "Belüften/Begasen" des Reaktorraumes über der Produktschmelze (Beispiel 3, 4). Dabei erhält man die Imidocarbonsäure PAC fast wasserfrei innerhalb von 30 Minuten bzw. eine 70 %ige Reduktion des Wassergehaltes nach nur dreimaligem "Belüften/Begasen". Der Phthalsäuregehalt läßt sich durch Überströmen mit Stickstoff immerhin um über 40 % reduzieren.

**Patentansprüche**

1. Verfahren zur Herstellung von gereinigten Phthalimidoalkancarbonsäuren, die mit Phthalsäure, Lactam und/oder Wasser verunreinigt sind, umfassend die Maßnahmen:
   a) Aufschmelzen der verunreinigten Phthalimidoalkancarbonsäure,
   b) Halten der Schmelze für eine so lange Zeit und bei einer solchen Temperatur, so daß sich die Verunreinigungen im wesentlichen aus der Schmelze entfernt haben und
   c) Abkühlen der gereinigten Produktschmelze.

2. Verfahren nach Anspruch 1, dadurch gekennzeichnet, daß in Schritt a) die Temperatur der Schmelze zwischen 110 und 200°C, bevorzugt zwischen 130 und 170°C liegt.

3. Verfahren nach Anspruch 1 oder 2, dadurch gekennzeichnet, daß das Aufschmelzen in Schritt a) unter Begasung mit einem Inertgas erfolgt.

4. Verfahren nach einem der Ansprüche 1 bis 3, dadurch gekennzeichnet, daß die Schmelze in Schritt b) bei vermindertem Inertgasdruck gehalten wird.

5. Verfahren nach einem der Ansprüche 1 bis 4, dadurch gekennzeichnet, daß in Schritt b) der Inertgasdruck 0,1 bis 300 mbar, bevorzugt 1 bis 140 mbar, besonders bevorzugt 4 bis 95 mbar beträgt.

6. Verfahren nach einem der Ansprüche 1 bis 5, dadurch gekennzeichnet, daß der Zeitraum in Schritt b) bis zu 5 Stunden beträgt.

6

7. Verfahren nach Anspruch 1, dadurch gekennzeichnet, daß in Schritt a) die verunreinigte Phthalimidoalkancarbonsäure in einer druckdichten Apparatur nach dem Evakuieren der Apparatur mit einem Inertgas begast wird und in der geschlossenen Apparatur geschmolzen wird und in Schritt b) der entstehende Überdruck entspannt wird, nach dem Entspannen erneut Inertgas in die Apparatur eingeleitet wird, die Apparatur druckdicht verschlossen wird und nach Einstellen eines Überdruckes wieder entspannt wird und dieser Vorgang solange wiederholt wird, bis sich kein Überdruck mehr bildet.

8. Verfahren nach Anspruch 1, dadurch gekennzeichnet, daß in Schritt a) die verunreinigte Phthalimidoalkancarbonsäure mit einem Inertgas begast wird und anschließend oder gleichzeitig geschmolzen wird und in Schritt b) die Schmelze mit einem Inertgas begast wird.

9. Verfahren nach Anspruch 1 oder 8, dadurch gekennzeichnet, daß das Begasen der Schmelze durch Überleiten eines Inertgasstromes erfolgt.

10. Verfahren nach einem der Ansprüche 1, 8 und 9, dadurch gekennzeichnet, daß die Strömungsgeschwindigkeit des Inertgasstromes 0,1 bis 50 l/h, bevorzugt 1 bis 30 l/h, besonders bevorzugt 2 bis 20 l/h, beträgt.

11. Verfahren nach einem der Ansprüche 1, 8 bis 10, dadurch gekennzeichnet, daß das Inertgas bei Normaldruck oder Unterdruck, bevorzugt im Bereich von 500 - 800 mbar über die Schmelze geleitet wird.

12. Verfahren nach einem der Ansprüche 1, 8 bis 11, dadurch gekennzeichnet, daß der Inertgasstrom über einen Zeitraum von 0,1 bis 6 Stunden, bevorzugt 0,3 bis 3 Stunden, besonders bevorzugt 0,5 bis 1,5 Stunden über die Schmelze geleitet wird.

13. Verfahren nach einem der Ansprüche 1 bis 12, dadurch gekennzeichnet, daß die gereinigte Produktschmelze bis zum Festpunkt oder bis unmittelbar oberhalb des Festpunktes abgekühlt wird.

Europäisches
Patentamt

EUROPÄISCHER RECHERCHENBERICHT

Nummer der Anmeldung

EP 94 10 0196

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | US-A-3 210 313 (ALLIED CHEMICAL CORPORATION) <br> * Spalte 3, Beispiel A ; Spalte 4, Zeile 5-25 * <br> --- | 1-13 | C07D209/48 |
| D,A | EP-A-0 349 940 (HOECHST AG) <br> * die Herstellung der Imidocarbonsäuren * <br> ----- | 1 | |

| | | | RECHERCHIERTE SACHGEBIETE (Int.Cl.5) |
|---|---|---|---|
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 13. April 1994 | Van Bijlen, H |